# EUROPÄISCHE PATENTSCHRIFT

(11) **EP 3 513 828 B1**
(45) Veröffentlichungstag und Bekanntmachung des Hinweises auf die Patenterteilung: **24.02.2021**
(21) Anmeldenummer: 18000930.0
(22) Anmeldetag: 14.11.2013
(51) Int. Cl.: A61M 16/06

(54) **MASKENWULST FÜR EIN PATIENTENINTERFACE**
MASK BEAD FOR A PATIENT INTERFACE
BOURRELET DE MASQUE POUR UNE INTERFACE PATIENT

(30) Priorität: 29.11.2012 DE 102012023268
(43) Veröffentlichungstag der Anmeldung: 24.07.2019
(62) Teilanmeldung aus: 13005359.8
(73) Patentinhaber: Löwenstein Medical Technology S.A., 2557 Luxembourg (LU)
(72) Erfinder: Eifler, Martin, 25348 Glückstadt (DE)
(74) Vertreter: Marx, Thomas

(56) Entgegenhaltungen:
- EP-A2- 1 147 782
- EP-A2- 2 732 839
- WO-A1-2010/135785
- WO-A1-2013/027144
- WO-A1-2013/186654
- US-A1- 2008 110 464
- US-A1- 2009 145 429

## Beschreibung

Masken werden im Bereich der Beatmung und der Schlaftherapie eingesetzt, wobei ein Patient mittels einer Flow- oder Druckquelle mit Atemgas versorgt wird. Die Maske stellt die Schnittstelle zwischen Patient und Gerät dar. Sie besteht meistens aus einem Maskenkörper mit einem Maskenwulst und wird mittels einer Bänderung und evtl. einer zusätzlichen Stirnabstützung am Kopf des Patienten fixiert. Der Maskenkörper besteht in der Regel aus einem relativ steifen Kunststoff und der Maskenwulst, der am Gesicht, bzw. an Gesichtspartien des Patienten anliegt, besteht aus einem relativ dünnen, weichen Material, vorzugsweise aus einem hautfreundlichen Silikon, einem thermoplastischen Elastomer (TPE) oder aus Polyurethan und besitzt in der Regel eine Dichtlippe, um eine ausreichende Abdichtung zu gewährleisten und Leckagen zu verhindern.

Herkömmliche Maskenwülste für eine Beatmungsmaske gibt es meistens üblicherweise in den Größen S, M und L, um möglichst viele verschiedene Gesichts- bzw. Nasen-Beschaffenheiten der Patienten zu berücksichtigen und einen möglichst hohen Trage- und Passkomfort und eine hohe Dichtigkeit zu erzielen.

Derartig bekannte Lösungen haben zur Folge, dass die Anzahl der anzubietenden Masken groß ausfallen muss, um alle verschiedenen Gesichtsformen und Größen abzudecken.

Die US 2008/0110464 A1 zeigt ein Kissen für ein Patienteninterface mit einer Basiswand, die mit einem Rahmen verbunden ist, ein darunterliegendes Stützkissen, welches sich von der Basiswand in Richtung des Gesichtes erstreckt, und eine Membran, die mindestensein Teil des darunterliegenden Kissens abdeckt. Das darunterliegende Kissen und/oder die Basiswand definieren eine Federkonstante, die entlang einer Länge der Dichtung variiert.

Die vorliegende Erfindung stellt sich zur Aufgabe einen Maskenwulst für Nasalmasken und/oder Vollgesichtsmasken zu entwickeln, der eine große Anpassungsfähigkeit an verschieden große und verscheiden geformte Gesichts- und Nasenpartien der Patienten aufweist. Patienten können verschiedene Nasenformen mit hohen oder niedrigen Nasenrückenregionen, schmale oder breite sowie lange oder kurze Nasen haben. Der Maskenwulst soll deshalb in der Lage sein, sich allen möglichen Nasenformen anzupassen und die gewünschte Passform und Dichtigkeit zu erzielen.

Die vorliegende Erfindung betrifft einen Maskenwulst, wie von beigefügtem Anspruch 1 definiert. Weitere Aspekte von dem Maskenwulst nach der Erfindung sind in abhängigen Ansprüche 2-12 definiert.

Um einen großen Bereich an unterschiedlichsten Nasengrößen und -formen abzudecken, wird ein Maskenwulst vorgeschlagen, der von der Länge im Größenbereich einer Maske der Größe S und von der Breite im Größenbereich einer Maske der Größe M liegt. Daraus ergibt sich ein Verhältnis von Länge (L) zu Breite (B2) des Maskenwulstes welches ungefähr 1:1 ist. Zum Anknüpfen an den Maskenkörper dient beispielsweise ein umlaufendes u-förmiges Halteprofil als Kontaktbereich, welches einen Innenschenkel und einen Außenschenkel mit einem Hinterschnitt aufweist und über einer umlaufenden Kante am Maskenkörper einrastet. Zur genauen Positionierung dienen Querstege im Halteprofil des Maskenwulstes, die in Nuten im Maskenkörper eingefügt werden müssen. Möglich ist es aber auch, den Maskenwulst im Kontaktbereich mittels 2K-Verfahren mit dem Maskenkörper zu verbinden.

Um eine hohe Anpassungsfähigkeit an verschieden große und verschieden geformte Nasenpartien eines Patienten zu erzielen, weist der Maskenwulst im Nasenrückenbereich eine sehr breite Form (B1) mit einem sehr großen, dünnwandigen Bereich auf, der sich leicht verformen lässt.

Die Anpassungsfähigkeit an verschieden große und verschieden geformte Nasenpartien der Patienten zeigt sich insbesondere in der besonderen Ausführung des Nasenrückenbereichs. Aus dem Vergleich der Erfindung mit dem Stand der Technik wird deutlich, dass bei dem erfindungsgemäßen Wulst neben dem Verhältnis B1 zu B2 auch das Verhältnis H1 zu H2 vom Stand der Technik abweicht. Erfindungsgemäß ist H1 immer gleichgroß oder größer als H2. Bei Masken aus dem Stand der Technik ist H1 immer kleiner als H2.

Die Erfindung ist anwendbar für elastische Maskenwülste, die auf einen harten Maskenkörper lösbar oder dauerhaft befestigt sind, und ebenso für Maskenwülste, die vollständig aus einem elastischen Material gefertigt sind. Maskenwülste, die vollständig aus einem elastischen Material gefertigt sind, weisen dann statt dem harten Maskenkörper entweder einen harten Maskenrahmen auf, in den der Maskenwulst eingefügt ist, oder weisen einen verdickten oder versteiften Abschnitt auf, die die Stützfunktion des harten Maskenkörpers übernimmt. Der Kontaktbereich zum Maskenkörper ist dann regelmäßig die Region, wo der elastische Maskenwulst in einen verdickten oder versteiften Abschnitt übergeht oder von dem Maskenrahmen gestützt wird.

Der Maskenwulst ist aus einem angenehmen hautfreundlichen Material, bevorzugt aus Silikon in einem Shore-Härtebereich zwischen 35 bis 55 Shore A, bevorzugt 40 - 50 Shore A.

Der Maskenwulst kann im 2K-Verfahren hergestellt sein, wobei der Wulst aus einem weichen Material hergestellt wird und bevorzugt an ein hartes Material angespritzt wird. Das harte Material definiert den Kontaktbereich oder der Maskenkörper oder ein Teil des Wulstes mit Stützfunktion. Im letztgenannten Fall ist der Wulst zweiteilig aus einem weichen und einem harten Material hergestellt. Der Kontakt wird bevorzugt über eine Haftung der Materialien erreicht, alternativ oder zusätzlich können mechanische Kontaktstellen die Haftung verstärken (Formschluss).

Ein beispielhafter Wulst kann auch einen Hohlraum aufweisen, der ein federnd nachgiebiges Füllmaterial aufweist. Der Hohlraum ist bevorzugt umlaufend ausgestaltet, in einem alternativen Beispiel ist im Nasenrückenbereich kein gefüllter Hohlraum vorgesehen. Das Füllmaterial kann ein Gel oder Schaum oder Gas oder eine Flüssigkeit oder ein weicher Füllstoff sein. Ein Gel würde im flüssigen Zustand in den Hohlraum eingefüllt werden und dort aushärten. Das Füllmaterial weist bevorzugt eine Härte im Bereich 2 Shore 000 bis 20 Shore 00, bevorzugt im Bereich 2 Shore 00 bis 10 Shore 00, besonders bevorzugt im Bereich 10 Shore 00 bis 20 Shore 00. Die Füllung kann auch unterschiedlich hart ausgeführt werden. Gesichtsnah weicher und Gesichtsfern härter.

Der Maskenwust ist bevorzugt aus Silikon oder PU hergestellt. Das Material des Maskenwustes weist eine Dehnbarkeit auf, die im Bereich über 500 % liegt, bevorzugt bei über 800 %, besonders bevorzugt über 1000 %.

### Figurenbeschreibung

- Fig.1a:: Maskenwulst (M) in Draufsicht
- Fig.1b:: Maskenwulst (M) in Seitenansicht
- Fig.1c:: Maskenwulst (M) von oben
- Fig.2a:: vertikale Schnittdarstellung des Maskenwulstes (M) entlang der Ebene x/x
- Fig.2b:: horizontale Schnittdarstellung des Maskenwulstes (M) entlang der Ebene y/y
- Fig.2c:: .horizontale Schnittdarstellung des Maskenwulstes (M) entlang der Ebene z/z
- Fig.3:: Vergleich Erfindung zum Stand der Technik Perspektivdarstellungen von Maskenwülsten (M), (M') und (M")
- Fig.4:: Maskenwulst (M') in verschiedenen Ansichten
- Fig.5:: Schnittdarstellungen des Maskenwulstes (M')
- Fig.6:: Maskenwulst (M") in verschiedenen Ansichten
- Fig.7:: Schnittdarstellungen des Maskenwulstes (M")

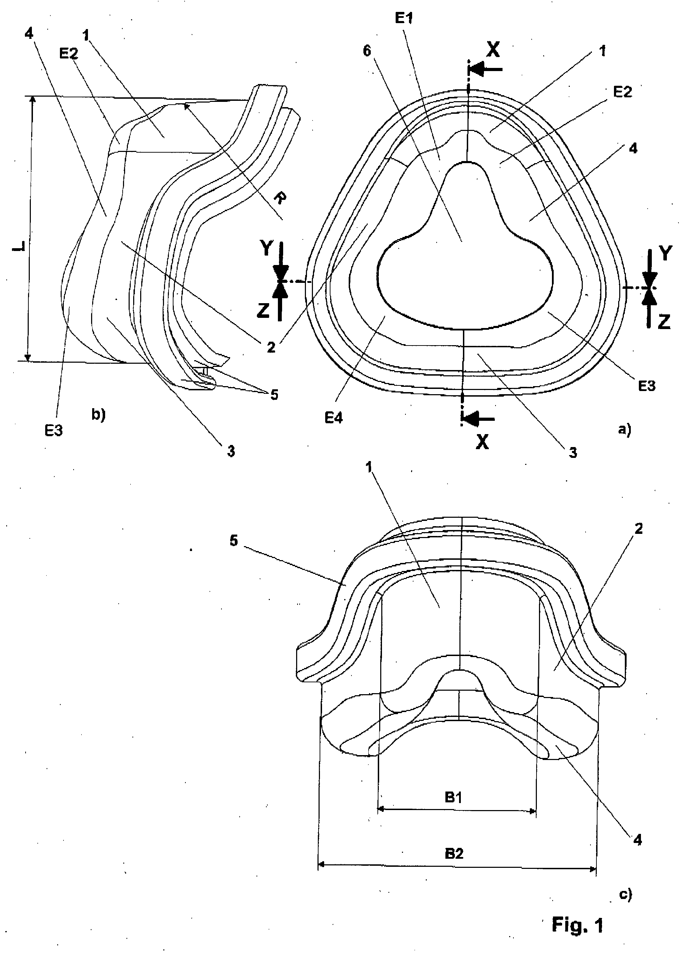

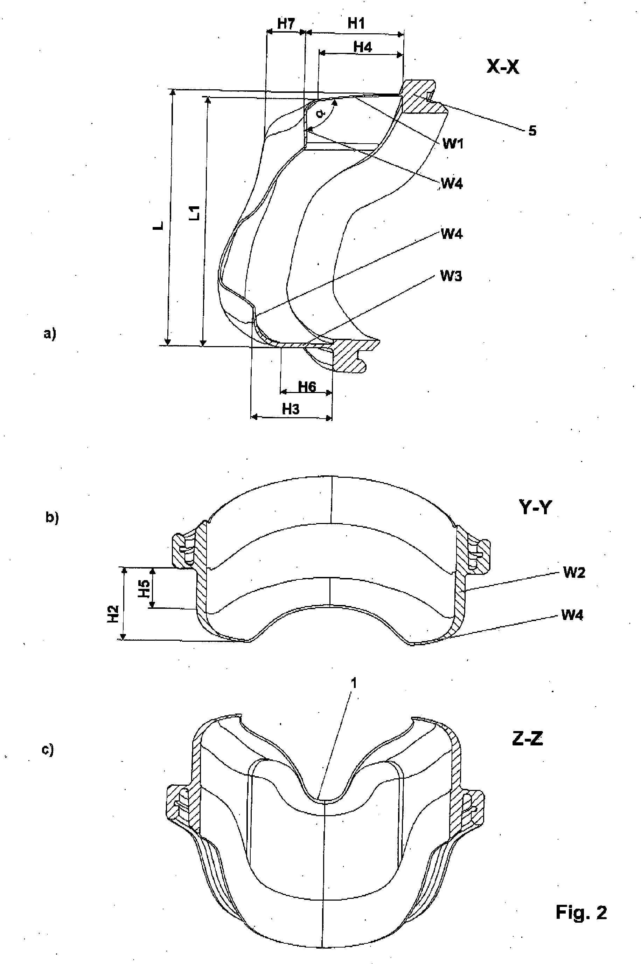

Die Figur 1a zeigt den Maskenwulst in der Draufsicht. Eingezeichnet ist eine vertikale Schnittebene x und eine horizontale Schnittebene y/z. Die Schnitte sind in Figur 2 veranschaulicht. Der Maskenwulst weist einen Nasenrückenbereich (1), Seitenbereiche (2), den Basisbereich (3) und den Auflagebereich (4) zum Patienten auf. Die Seitenbereiche (2) erstrecken sich ausgehend vom Kontaktbereich zum Maskenkörper (5) im Wesentlichen vertikal nach oben. Der Auflagebereich (4) zum Patienten ist als eine Einzeldichtlippe ausgeführt, die den anderen Bereichen angeformt ist und innen eine Öffnung (6) freigibt, die der Einführung zumindest der Nase des Patienten dient. Der Auflagebereich (4) erstreckt sich ausgehend von den Seitenbereichen (2) und dem Basisbereich (3) sowie dem Nasenrückenbereich (1) zur Öffnung hin gebogen, wobei sich der Auflagebereich (4) zumindest teilweise horizontal zum Seitenbereich (2) erstreckt.

Die Figur 1b zeigt den Maskenwulst in der Seitenansicht. Im Nasenrückenbereich (1) ist der Patient am empfindlichsten, daher wird hier eine sehr dünne Wand (W1) von ≤ 0,6 mm, bevorzugt, im Bereich 0,25 - 0,5 mm gewählt. Der Nasenrückenbereich lässt sich leicht verformen und der Nasenform anpassen. Um ein Einknicken dieses Bereiches bei Druck auf den Bereich zu vermeiden, wurde in Gegensatz zu den Bereichen (2 und 3), die eine gerade Kontur aufweisen, eine bogenförmige Kontur (R) gewählt. Der Bogen im Nasenrückenbereich erstreckt sich vom Kontaktbereich zum Maskenkörper (5) hin zur Auflagefläche (4). Möglich ist aber auch, die Kontur (R) linear verlaufen zu lassen.
In Figur 1b ist ebenfalls zu erkennen, dass der Wulst eine Kontaktstelle zum Patientengesicht im Bereich des dünnwandigen Nasenrückenbereiches aufweist und eine dünnwandige Oberlippenauflage die durch einen seitlichen Stützbereich verbunden sind. An den Übergängen ergeben sich dominante Eckpunkte die der Ausrichtung der Maske auf dem Gesicht dienen. Die unteren Eckpunkte (E3, E4) befinden, beiderseits der Symmetrieebene und dienen bevorzugt der Ausrichtung der Maske an der Nasolabial Falte. Die oberen Eckpunkte. (E1, E2) liegen seitlich der Nase (im Bereich der Maxilla neben dem Nasenbein) und definieren das Ende des Stützbereiches und somit den Übergang zum Nasenrückenbereich mit der Breite (B1).

Figur 1c zeigt den Maskenwulst in der Ansicht von oben. Die Breite des Nasenrückenbereiches (B1) wurde breiter als bei bekannten Masken gewählt, um eine optimale Passform und Dichtigkeit auch bei breiteren und auch flachen Nasenformen zu gewährleisten. Durch die gewählte Geometrie sitzt der Maskenwulst tiefer auf der Nase des Anwenders. Ein hoher Nasenrücken taucht bei der gewählten Kontur tiefer in den Maskenwulst ein, was aber keinen Nachteil darstellt.

Um einen großen Bereich an unterschiedlichsten Nasengrößen und -formen abzudecken, wird ein Maskenwulst mit einer Länge (L) im Größenbereich von 50 mm bis 65 mm, bevorzugt 53 bis 62 mm, besonders bevorzugt 58 mm, und von einer Breite (B2) im Größenbereich 50 mm bis 65 mm, bevorzugt 53 bis 63 mm, besonders bevorzugt 59 mm, vorgeschlagen. Daraus ergibt sich ein Verhältnis von Länge (L) zu Breite (B2) von ungefähr 1:1.

Die Länge (L) liegt im Größenbereich einer handelsüblichen Maske der Größe S, wogegen die Breite (B2) im Größenbereich einer Maske der Größe M liegt. Die gesamten Proportionen der Maske sind dadurch im Vergleich zum Stand der Technik verändert worden.

Der Nasenrückenbereich (1) ist im Vergleich zum Stand der Technik wesentlich breiter, die Breite (B1) ist im Bereich 27 - 37 mm, bevorzugt bei 31 bis 35 mm, besonders bevorzugt bei 32,5 bis 35 mm. Der Nasenrückenbereich (1) kennzeichnet sich bevorzugt durch einen ersten Radius starker, bevorzugt konstanter, Krümmung, der im Seitenbereich in einen zweiten Radius mit weniger ausgeprägter Krümmung oder einen im Wesentlichen geraden Bereich (ohne Krümmung) , übergeht. Bevorzugt ist die Krümmung im Seitenbereich halb so groß oder kleiner als im Nasenrückenbereich. Der Nasenrückenbereich (1) ist im Vergleich zum Stand der Technik dünnwandiger mit einer Wandstärke (W1) ≤ 0,6 mm, bevorzugt 0,25 bis 0,5 mm.

Die Figur 2a zeigt den Wulst in der Ebene x geschnitten. Im Nasenrückenbereich (1) ist der Patient am empfindlichsten, daher wird hier eine sehr dünne Wand (W1) von ≤ 0,6 mm, bevorzugt 0,2 - 0,5 mm, besonders bevorzugt 0,25 mm gewählt. Dieser Bereich lässt sich leicht verformen und der Nasenform anpassen. Um ein Einknicken dieses Bereiches bei Druck auf den Bereich zu vermeiden, wurde in Gegensatz zu den Bereichen (2 und 3), die eine gerade Kontur aufweisen, eine bogenförmige Kontur (R), vom Kontaktbereich zum Maskenkörper (5) hin zur Auflagefläche (4) im Nasenrückenbereich, gewählt. Der Winkel α, gemessen von der Vertikalen (hier durch W4 veranschaulicht) zur Oberfläche der bogenförmigen Kontur (R), liegt daher bevorzugt im Bereich größer als 90°, besonders bevorzugt bei 91° bis 100°: Möglich ist aber auch die Kontur (R) linear verlaufen zu lassen, der Winkel α ist dann 90°.
Der Basisbereich (3), mit der Wandstärke (W3), ist im Gegensatz zu den Seitenbereichen (2), mit den Wandstärken (W2), ebenfalls dünn ausgeführt, da ein Druck auf den Kiefer als unangenehm empfunden wird. Die Wandstärke (W3) beträgt im Bereich 0,8 - 1,2 mm, bevorzugt 1,0 mm. Da der Patient an den Kontaktstellen im Seitenbereich (2) des Maskenwulstes am unempfindlichsten ist, können die Wandstärken (W2) dieses Bereiches dicker ausgeführt sein und sorgen so für die Steifigkeit und Stützkraft des Maskenwulstes. Die Wandstärke (W2) beträgt in diesem Bereich 1,5 - 2,8 mm, bevorzugt 2,1 mm. Der Auflagebereich (4) zum Patienten ist als eine Einzeldichtlippe ausgeführt. Der Auflagebereich (4) wurde auf dem kompletten Umfang mit einer annähernd gleichbleibenden, dünnen Wandstärke (W4) von 0,2 - 0,75 mm, bevorzugt 0,5 mm, ausgebildet, die dem Patienten einen angenehmen Tragekomfort ermöglicht und zudem als Dichtlippe dient.

Maskenwülste, die aus dem Stand der Technik bekannt sind, weisen eine Doppellippe auf, wobei die innere Lippe als Stützlippe und die äußere Lippe als Dichtlippe ausgeführt ist. Die Wandstärken der einzelnen Bereiche (1 - 4) wurden erfindungsgemäß auf die Empfindlichkeit der Kontaktstellen im Gesicht des Anwenders angepasst. Durch die erfindungsmäßige Wahl von unterschiedlichen Wandstärken (W1-W4) der Bereiche (1-4) des Maskenwulstes kann auf eine zusätzliche Stützlippe verzichtet werden.
Die Höhe (H1) im Nasenrückenbereich beträgt 18 - 27 mm, bevorzugt 20 - 25 mm, besonders bevorzugt 22,2 mm und ist höher als bei Masken aus dem Stand der Technik. Dadurch wird ein weiter Bereich mit guten Dichteigenschaften für unterschiedliche Nasenrücken eröffnet. Die Höhe (H4) im Nasenrückenbereich ist höher als bei handelsüblichen Maskenwülsten und beträgt im Bereich 14 - 23 mm, bevorzugt 18,9 mm. Die Höhe (H3) des Basisbereiches (3) ist geringer als bei handelsüblichen Maskenwülsten und beträgt im Bereich 16 - 21 mm, bevorzugt 18,5 mm. Die Höhe (H6) des Basisbereiches (3) ist ebenfalls geringer als bei handelsüblichen Maskenwülsten und beträgt im Bereich 8,5 - 13,5 mm, bevorzugt 11,9 mm und hat eine Stützfunktion. Die Höhe (H7) (Differenz zwischen der Nasenrückenauflage und der Auflage im Seitenbereich) entspricht im Wesentlichen den Höhen der Masken aus dem Stand der Technik.

Die Figur 2b zeigt den Wulst in der Ebene y geschnitten. Die Höhe (H2) des Seitenbereiches (2) ist geringer als bei handelsüblichen Maskenwülsten und beträgt im Bereich 12 - 22 mm, bevorzugt 15,8 mm. Die Höhe (H5) des Seitenbereiches (2) ist geringer als bei handelsüblichen Maskenwülsten und beträgt im Bereich 6 - 14 mm, bevorzugt 8,4 mm. Zur erkennen ist zudem, wie die Wanddicke im Seitenbereich von der Basis (W2) hin zum Auflagebereich (W4) kontinuierlich abnimmt.

Die Figur 2c zeigt den Wulst in der Ebene z geschnitten. Hier erkennt man gut, dass der Nasenrückenbereich (1) vertieft ist.

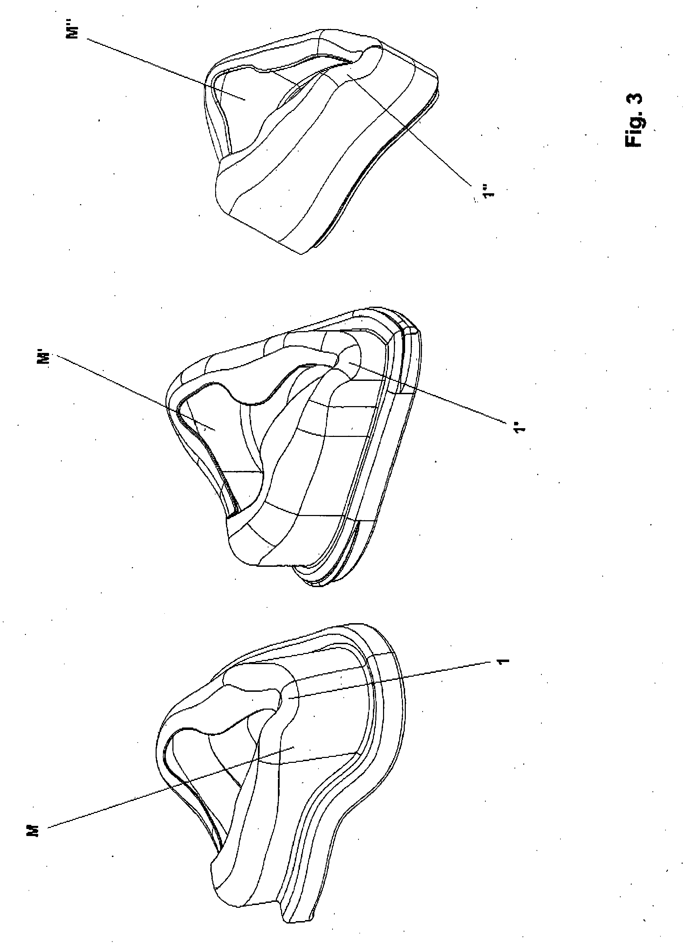

In der Figur 3 werden die Unterscheide des erfindungsmäßigen Maskenwulstes (M) zu Maskenwülsten aus dem Stand der Technik (M') und (M") in der Perspektivdarstellung deutlich. Insbesondere ist gut erkennbar, dass der Nasenrückenbereich (1) höher ist als die Nasenrückenbereiche (1' und 1'').

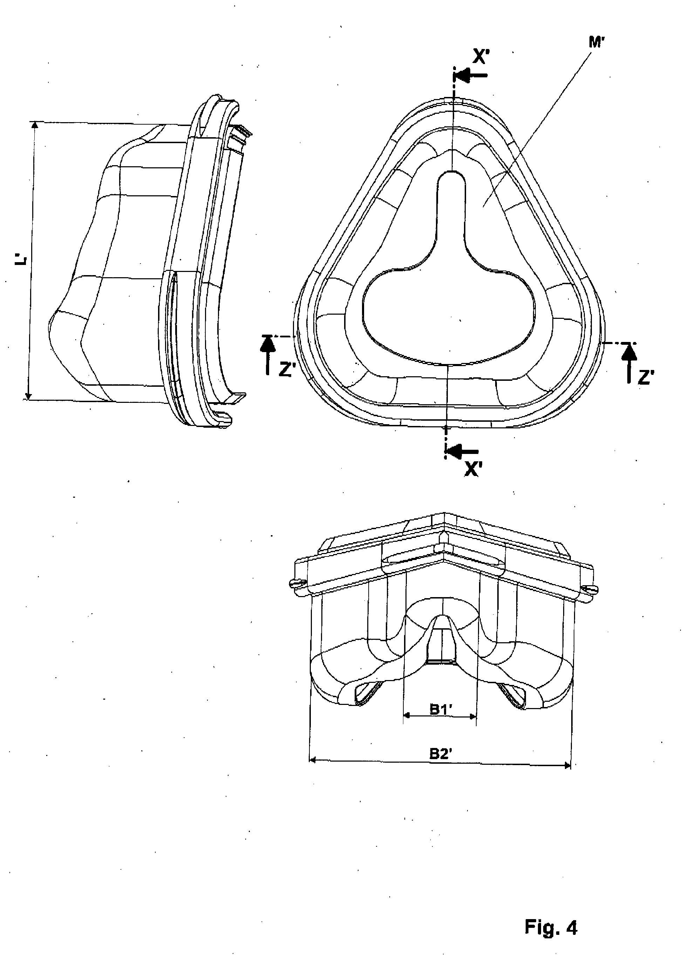

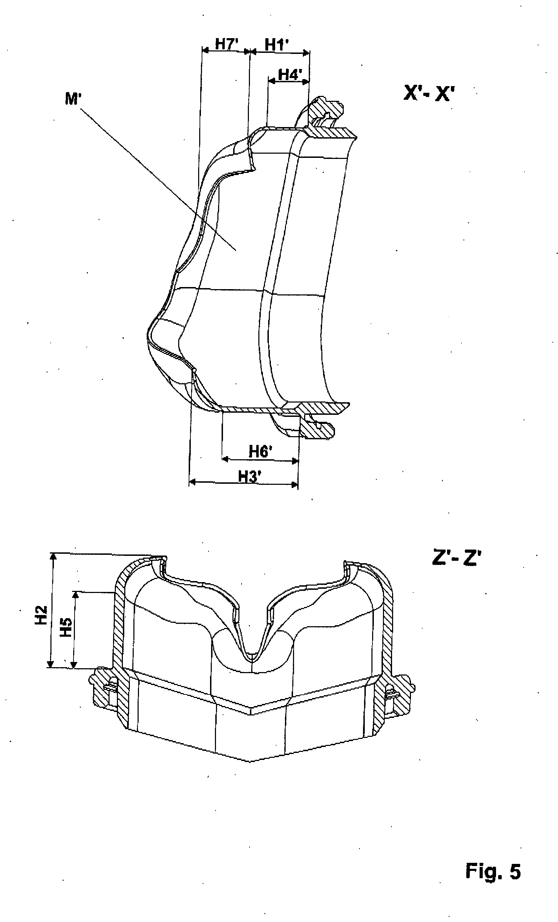

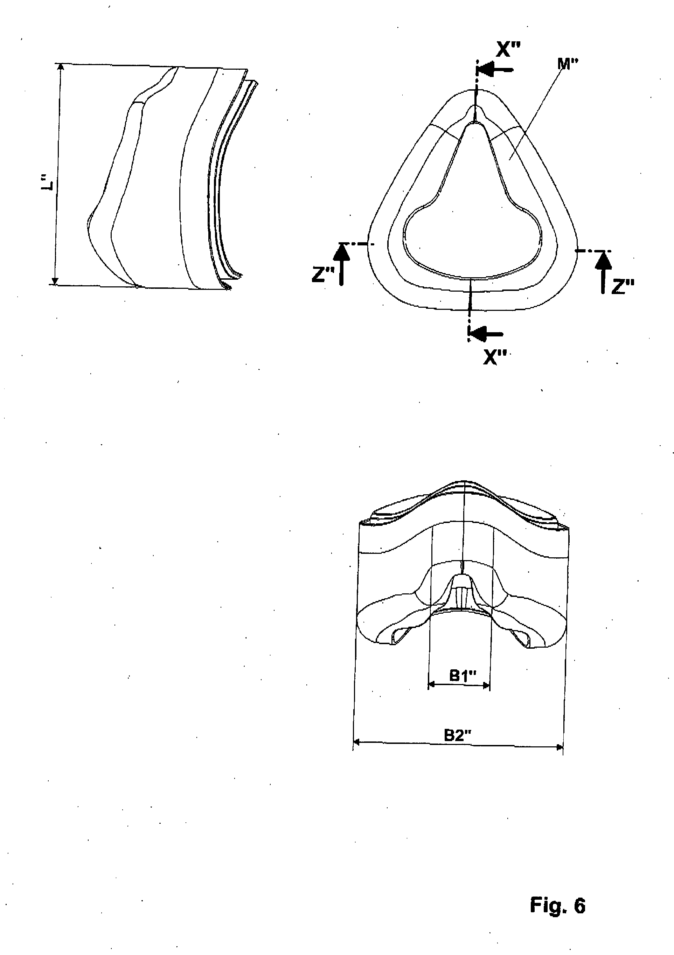

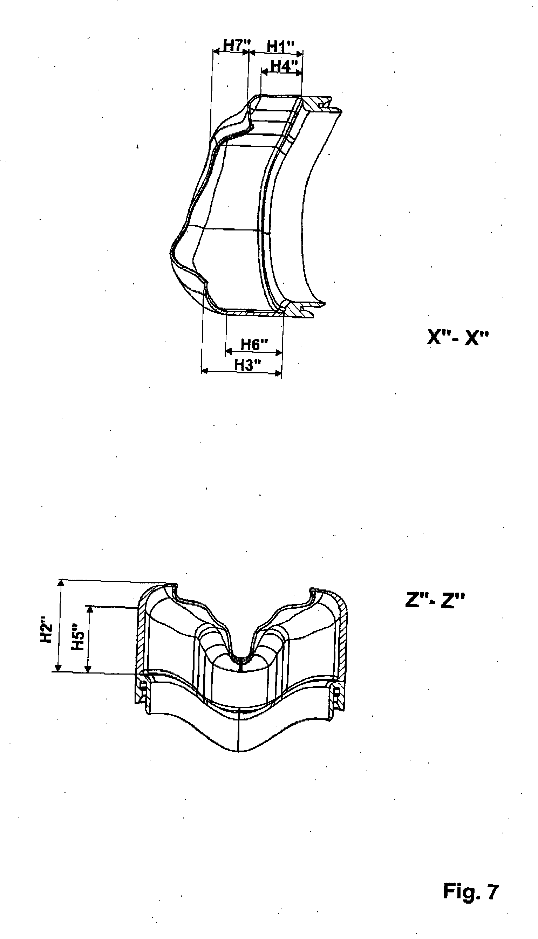

Die Figuren 4 und 5 zeigen einem Maskenwulst (M') und die Figuren 6 und 7 einen weiteren Maskenwulst (M") aus dem Stand der Technik.

Eine Gegenüberstellung der Masse des Maskenwulstes (M) zu den Massen der Maskenwülste (M' und M") macht den Unterscheid der Erfindung zum Stand der Technik deutlich.

Das maßgebliche Merkmal des erfindungsgemäßen Maskenwulstes (M) ist der sehr große Verformbereich des Nasenrückenbereiches (1), der durch die geeignete Wahl der Wandstärke (W1) und der Breite (B1) und der ersten Höhe (H1) und der weiteren Höhe (H4) erzielt wird. Dieser Bereich ist ausschlaggebend für die Anpassungsfähigkeit an verschieden große und verschieden geformte Nasenpartien der Patienten.
Die Breite (B2) ist die maximale Breite des Maskenwulstes gemessen an den Außenkanten der Seitenbereiche (2) im Bereich neben der Kontaktstelle (5) zum Maskenkörper. Die Breite (B2) ist alternativ die maximale Breite des Kontaktbereiches.
Die Länge (L) ist die maximale Länge des Maskenwulstes gemessen in der Symmetrieebene der Maske zwischen Nasenrückenbereich und Oberlippenbereich.
Die Anpassungsfähigkeit an verschieden große und verschieden geformte Nasenpartien der Patienten zeigt sich insbesondere in der besonderen Ausführung des Nasenrückenbereichs. Aus dem Vergleich der Erfindung mit dem Stand der Technik (siehe Figuren 3 bis 7) wird deutlich, dass bei dem erfindungsgemäßen Wulst neben dem Verhältnis B1 zu B2 auch das Verhältnis H1 zu H2 vom Stand der Technik abweicht. Erfindungsgemäß ist H1 immer gleichgroß oder größer als H2. Bei Masken aus dem Stand der Technik ist H1 immer kleiner als H2.

Die Länge (L) beträgt bevorzugt 58 mm und ist im Vergleich zu den Längen (L') mit 61,5 mm und (L") mit 62,5 mm kürzer.
Die Breite (B2, B2' und B2") ist bei allen drei Wülsten gleich und beträgt im Bereich 59 mm; wohingegen die Breite des Nasenrückenbereiches (B1) im Verhältnis zu den Breiten (B1') und (B1") doppelt so breit ist und erfindungsgemäß im Bereich 33,7 mm beträgt. Die Wandstärke (W1) ist im Vergleich zu den Wandstärken (W1') und (W1") halb so dick und liegt im Bereich zwischen 0,25 mm und 0,5 mm. Die Höhe (H1) beträgt im Bereich 18 - 27 mm, hier 22,2 mm und ist im Vergleich zu den Höhen H1'=12,7 mm und H1"=15,2 mm, um etwa das 1,4- bis 2,1-fache höher.
Die Höhe (H2) der Seitenbereiche (2) beträgt etwa nur das 0,6fache der Höhen (H2' und H2"), die Höhe (H3) des Kinnbereiches (3) beträgt etwa das 0,7fache der Höhen (H3' und H3") und ist somit kleiner als bei handelsüblichen Maskenwülsten.
Die Höhe (H7) (Differenz zwischen der Nasenrückenauflage und der Auflage im Seitenbereich) ist in etwa gleich den Höhen (H7' und H7").

Um eine hohe Anpassungsfähigkeit an verschieden große und verschieden geformte Nasenpartien eines Patienten zu erzielen, weist der Maskenwulst im Nasenrückenbereich eine sehr breite Form (B1) mit einem sehr großen, dünnwandigen Bereich auf, der sich leicht verformen lässt. Der Maskenwulst weist in diesem Bereich, der etwa 1/3 des Umfanges ausmacht, eine Wandstärke von ≤ 0,6 mm auf und ist in diesem Bereich zudem um etwa das 2-fache höher als in den Seitenbereichen des Wulstes. Um aber ein Einknicken dieses Bereiches bei Druck auf den Bereich zu vermeiden, wurde eine bogenförmige Kontur vom Kontaktbereich hin zur Auflagefläche im Nasenrückenbereich gewählt.

Die Erfindung eignet sich besonders für Masken die nur die Nase des Anwenders überdecken; obwohl auch gute Ergebnisse mit erfindungsgemäßen Vollgesichtsmasken erzielt wurden, die Mund und Nase des Anwenders überdecken.

Der Maskenwulst weist einen Bereich auf der sich zu Aufbringung einer Kennzeichnung eignet. Bevorzugt ist der Bereich im Oberlippenbereich. Dieser kann beispielsweise bedruckt oder gelasert oder graviert sein.

## Patentansprüche

1. Maskenwulst für ein Patienteninterface bestehend aus einem Kontaktbereich (5) zu einem Maskenkörper, einem Nasenrückenbereich (1) mit einer Breite (B1), im Übergangsbereich zum Seitenbereich (2), Seitenbereichen (2), einem Basisbereich (3) und einem Auflagebereich (4) zum Patienten, wobei der Auflagebereich (4) sich ausgehend von den Seitenbereichen (2) und dem Basisbereich (3) sowie dem Nasenrückenbereich (1) zu einer Öffnung (6) hin erstreckt und wobei der Auflagebereich (4) als Dichtlippe ausgeführt ist, welche die zentrale Öffnung (6) umrandet, die zumindest der Einführung der Nase des Patienten dient, wobei der Maskenwulst eine maximale Breite (B2) aufweist und das Verhältnis der Breiten B1 zu B2 eins zu drei oder größer ist, wobei eine Höhe (H1) des Maskenwulstes im Nasenrückenbereich (1), gemessen von dem Kontaktbereich (5) zu dem Auflagebereich (4), immer gleichgroß oder größer als die Höhe (H2) des Maskenwulstes in dem Seitenbereich (2), gemessen von dem Kontaktbereich (5) zu dem Auflagebereich (4), ist, **dadurch gekennzeichnet, dass** die Höhe (H1) im Nasenrückenbereich (1) im Bereich 18 - 27 mm ist und dass die Höhe (H2) des Seitenbereiches (2) im Bereich 12 - 22 mm ist.

2. Maskenwulst nach Anspruch 1 **dadurch gekennzeichnet, dass** im Nasenrückenbereich (1) eine Wandstärke (W1) von ≤ 0,6 mm verwendet wird.

3. Maskenwulst nach Anspruch 1 **dadurch gekennzeichnet, dass** der Nasenrückenbereich (1) eine Breite (B1) im Bereich 27 - 37 mm aufweist.

4. Maskenwulst nach zumindest einem der vorhergehenden Ansprüche **dadurch gekennzeichnet, dass** die Höhe (H4), gemessen von dem Kontaktbereich (5) zu dem Übergang von Wandbereichen (W1) zu (W4), im Nasenrückenbereich im Bereich 14 - 23 mm ist.

5. Maskenwulst nach zumindest einem der vorhergehenden Ansprüche **dadurch gekennzeichnet, dass** der Nasenrückenbereich (1) eine bogenförmige Kontur (R) aufweist.

6. Maskenwulst nach zumindest einem der vorhergehenden Ansprüche **dadurch gekennzeichnet, dass** der Basisbereich (3) eine Wandstärke (W3) im Bereich 0,8 -1,2 mm aufweist und/oder dass der Seitenbereich (2) eine Wandstärke (W2) im Bereich 1,5 - 2,8 mm aufweist.

7. Maskenwulst nach zumindest einem der vorhergehenden Ansprüche **dadurch gekennzeichnet, dass** die Wanddicke im Seitenbereich von der Basis (W2) hin zum Auflagebereich (4) hin kontinuierlich abnimmt.

8. Maskenwulst nach zumindest einem der vorhergehenden Ansprüche **dadurch gekennzeichnet, dass** der Auflagebereich (4) eine annähernd gleichbleibende Wandstärke (W4) im Bereich 0,2 - 0,75 mm aufweist.

9. Maskenwulst nach zumindest einem der vorhergehenden Ansprüche, **dadurch gekennzeichnet, dass** der Auflagebereich (4) an den Übergängen Nasenrückenbereich (1) zum Seitenbereich (2) und Basisbereich zum Seitenbereich Eckpunkte (E1 - E4) aufweist die der Ausrichtung der Maske auf dem Gesicht dienen.

10. Maskenwulst nach dem vorhergehenden Anspruch, **dadurch gekennzeichnet, dass** die unteren Eckpunkte (E3, E4) sich beiderseits der Symmetrieebene (x-x) befinden und der Ausrichtung des Wulstes an der Nasolabial-Falte dienen.

11. Maskenwulst nach zumindest einem der beiden vorhergehenden Ansprüche **dadurch gekennzeichnet, dass** die oberen Eckpunkte (E1, E2) seitlich der Nase im Bereich liegen und definieren das Ende eines Stützbereiches und somit den Übergang zum Nasenrückenbereich mit der Breite (B1).

12. Maskenwulst nach zumindest einem der vorhergehenden Ansprüche **dadurch gekennzeichnet, dass** das Material des Maskenwustes eine Dehnbarkeit aufweist, die über 800 % liegt.

## Claims

1. A mask bead for a patient interface, consisting of a contact area (5) to a mask body, a nose bridge area (1) having a width (B1), in the transition area to the side area (2), side areas (2), a base area (3) and a bearing area (4) on the patient, wherein the bearing area (4) extends from the side areas (2) and the base area (3) and the nose bridge area (1) to an opening (6) and wherein the bearing area (4) is designed as a sealing lip which frames the central opening (6) which serves at least for the introduction of the nose of the patient, wherein the mask bead has a maximum width (B2) and the ratio of the widths B1 to B2 is one to three or greater, wherein a height (H1) of the mask bead in the nose bridge area (1), measured from the contact area (5) to the bearing area (4), is always the same as or greater than the height (H2) of the mask bead in the side area (2), measured from the contact area (5) to the bearing area (4), **characterized in that** the height (H1) in the nose bridge area (1) is in the range of 18 - 27 mm and the height (H2) of the side area (2) is in the range of 12 - 22 mm.

2. The mask bead according to Claim 1, **characterized in that** a wall thickness (W1) of ≤ 0.6 mm is used in the nose bridge area (1) .

3. The mask bead according to Claim 1, **characterized in that** the nose bridge area (1) has a width (B1) in the range of 27 - 37 mm.

4. The mask bead according to at least one of the preceding claims, **characterized in that** the height (H4), measured from the contact area (5) to the transition of wall areas (W1) to (W4), in the nose bridge area is in the range of 14 - 23 mm.

5. The mask bead according to at least one of the preceding claims, **characterized in that** the nose bridge area (1) has an arc-shaped contour (R).

6. The mask bead according to at least one of the preceding claims, **characterized in that** the base area (3) has a wall thickness (W3) in the range of 0.8 - 1.2 mm and/or the side area (2) has a wall thickness (W2) in the range of 1.5 - 2.8 mm.

7. The mask bead according to at least one of the preceding claims, **characterized in that** the wall thickness in the side area decreases continuously from the base (W2) toward the bearing area (4).

8. The mask bead according to at least one of the preceding claims, **characterized in that** the bearing area (4) hay an approximately constant wall thickness (W4) in the range of 0.2 - 0.75 mm.

9. The mask bead according to at least one of the preceding claims, **characterized in that** the bearing area (4) at the transitions from nose bridge area (1) to side area (2) and from base area to side area has corner points (E1 - E4) which serve to align the mask on the face.

10. The mask bead according to the preceding claim, **characterized in that** the lower corner points (E3, E4) are located on both sides of the plane of symmetry (x-x) and serve to align the bead on the nasolabial groove.

11. The mask bead according to at least one of the two preceding claims, **characterized in that** the upper corner points (E1, E2) lie in the area to the sides of the nose and define the end of a support area and, consequently, the transition to the nose bridge area with the width (B1).

12. The mask bead according to at least one of the preceding claims, **characterized in that** the material of the mask bead has an extensibility which is more than 800%.

## Revendications

1. Bourrelet de masque destiné à une interface patient comprenant une zone de contact (5) avec une coque de masque, une zone du dos de nez (1) d'une largeur (B1), dans la zone de transition à la zone latérale (2), des zones latérales (2), une zone de base (3) et une zone d'appui (4) sur le visage du patient, dans lequel la zone d'appui (4) s'étend en partant des zones latérales (2) et de la zone de base (3) ainsi que de la zone du dos de nez (1) vers une ouverture (6) et dans lequel la zone d'appui (4) est exécutée sous la forme d'une lèvre d'étanchéité, laquelle borde l'ouverture centrale (6), qui sert au moins à l'insertion du nez du patient, dans lequel le bourrelet de masque présente une largeur maximale (B2) et le rapport des largeurs B1 à B2 est de un pour trois ou supérieur, dans lequel une hauteur (H1) du bourrelet de masque dans la zone du dos de nez (1), mesurée de la zone de contact (5) avec la zone d'appui (4), est toujours aussi grande ou plus grande que la hauteur (H2) du bourrelet de masque dans la zone latérale (2), mesurée de la zone de contact (5) avec la zone d'appui (4), **caractérisé en ce que** la hauteur (H1) dans la zone du dos de nez (1) se situe dans la plage 18 - 27 mm et que la hauteur (H2) de la zone latérale (2) se situe dans la plage 12 - 22 mm.

2. Bourrelet de masque selon la revendication 1 **caractérisé en ce qu'**une épaisseur de paroi (W1) ≤ 0,6 mm est utilisée dans la zone du dos de nez (1).

3. Bourrelet de masque selon la revendication 1 **caractérisé en ce que** la zone du dos de nez (1) présente une largeur (B1) se situant dans la plage 27 - 37 mm.

4. Bourrelet de masque selon l'une au moins des revendications précédentes, **caractérisé en ce que** la hauteur (H4), mesurée de la zone de contact (5) avec la transition des zones de paroi (W1) à (W4), se situe dans la plage 14 - 23 mm dans la zone du dos de nez.

5. Bourrelet de masque selon l'une au moins des revendications précédentes, **caractérisé en ce que** la zone du dos de nez (1) présente un contour incurvé (R).

6. Bourrelet de masque selon l'une au moins des revendications précédentes, **caractérisé en ce que** la zone de base (3) présente une épaisseur de paroi (W3) se situant dans la plage 0,8 - 1,2 mm et / ou que la zone latérale (2) présente une épaisseur de paroi (W2) se situant dans la plage 1,5 - 2,8 mm.

7. Bourrelet de masque selon l'une au moins des revendications précédentes, **caractérisé en ce que** l'épaisseur de paroi dans la zone latérale diminue continuellement de la base (W2) jusqu'à la zone d'appui (4).

8. Bourrelet de masque selon l'une au moins des revendications précédentes, **caractérisé en ce que** la zone d'appui (4) présente une épaisseur de paroi quasiment constante (W4) se situant dans la plage 0,2 - 0,75 mm.

9. Bourrelet de masque selon l'une au moins des revendications précédentes, **caractérisé en ce que** la zone d'appui (4) au niveau des transitions entre la zone du dos de nez (1) et la zone latérale (2), et entre la zone de base et la zone latérale présente des points d'angle (E1 - E4) qui servent à orienter le masque sur le visage.

10. Bourrelet de masque selon la revendication précédente, **caractérisé en ce que** les points d'angle inférieurs (E3, E4) se trouvent de part et d'autre du plan de symétrie (x-x) et qu'ils servent à orienter le bourrelet au niveau de la fente nasolabiale.

11. Bourrelet de masque selon l'une au moins des deux revendications précédentes, **caractérisé en ce que** les points d'angle supérieurs (E1, E2) se trouvent sur les côtés du nez dans la zone et qu'ils définissent la fin d'une zone de support et ainsi la transition vers la zone du dos de nez de la largeur (B1) .

12. Bourrelet de masque selon l'une au moins des revendications précédentes, **caractérisé en ce que** le matériau du bourrelet de masque présente une extensibilité supérieure à 800 %.
